(19) Europäisches Patentamt
European Patent Office
Office européen des brevets

(11) Veröffentlichungsnummer : **0 168 732**
**B1**

(12) **EUROPÄISCHE PATENTSCHRIFT**

(45) Veröffentlichungstag der Patentschrift : **27.07.88**

(51) Int. Cl.⁴ : **C 07 C 69/738**, C 07 C 67/00

(21) Anmeldenummer : **85108397.2**

(22) Anmeldetag : **06.07.85**

(54) **Verfahren zur Herstellung von halogenierten Aroylessigestern.**

(30) Priorität : **18.07.84 DE 3426482**

(43) Veröffentlichungstag der Anmeldung : **22.01.86 Patentblatt 86/04**

(45) Bekanntmachung des Hinweises auf die Patenterteilung : **27.07.88 Patentblatt 88/30**

(84) Benannte Vertragsstaaten : **AT BE CH DE FR GB IT LI NL SE**

(56) Entgegenhaltungen :
**EP-A- 0 078 362**
**EP-A- 0 113 092**
**DE-A- 3 142 856**
**US-A- 3 818 072**
**CHEMICAL ABSTRACTS, Band 89, Nr. 21, 20. November 1978, Seite 528, Spalte 2, Zusammenfassungsnr. 179023x, Columbus, Ohio, US; E.C. TAYLOR et al.: "A convenient synthesis of beta-keto esters"**

(73) Patentinhaber : **BAYER AG**
**Konzernverwaltung RP Patentabteilung**
**D-5090 Leverkusen 1 Bayerwerk (DE)**

(72) Erfinder : **Grohe, Klaus, Dr.**
**Am Wasserturm 10**
**D-5068 Odenthal (DE)**

**EP 0 168 732 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von halogenierten Aroylessigsäureestern, die wertvolle Zwischenprodukte für die Synthese von hochwirksamen antibakteriellen Arzneimitteln sind.

Es wurde gefunden, daß man halogenierte Aroylessigester der Formel I

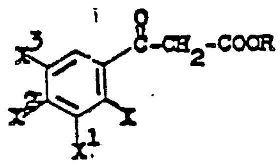

(I)

in welcher

R für Alkyl mit 1-6 Kohlenstoffatomen steht,

X für Halogen, bevorzugt Chlor oder Fluor steht,

$X^1$ Wasserstoff, Halogen, bevorzugt Fluor, bedeutet,

$X^2$ Halogen, bevorzugt Chlor oder Fluor, sein kann und

$X^3$ Wasserstoff oder Halogen, bevorzugt Fluor, darstellt, erhält, wenn man Benzoylchloride der Formel II

in welcher X, $X^1$, $X^2$ und $X^3$ die oben angegebene Bedeutung haben mit Dichlorethylen III in Gegenwart von wasserfreiem Aluminiumchlorid zu den Aryl-trichlorethylketonen IV umsetzt, diese unter Abspaltung von Chlorwasserstoff in die Aryl-dichlorvinylketone V überführt und letztere mit Alkoholat und anschließend mit einer wäßrigen Säure behandelt.

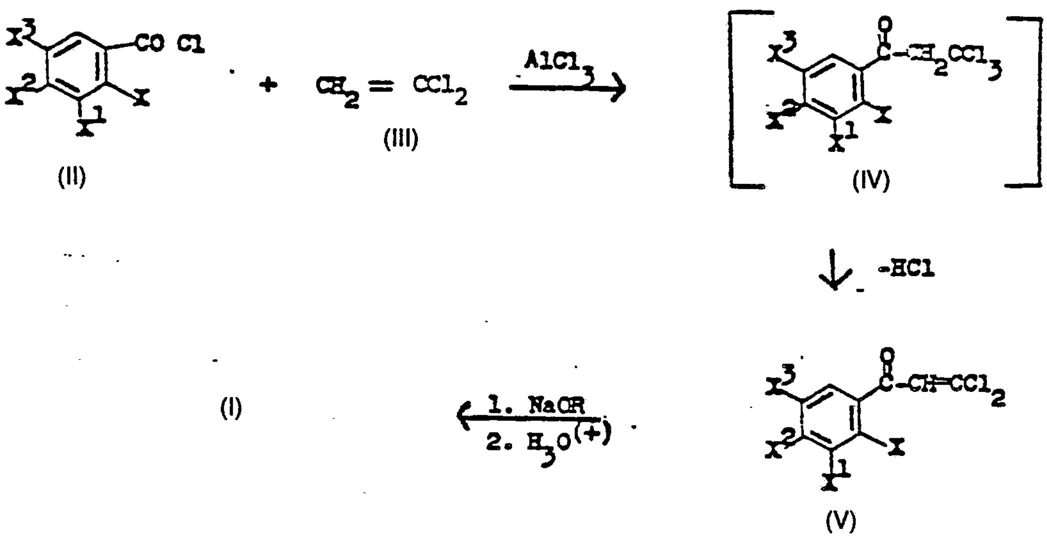


(II) (III) (IV) (I) (V)

Das erfindungsgemäße Verfahren weist eine Reihe von Vorteilen auf. So kann man auf eine Reinigung der Ketone IV verzichten und die Rohprodukte direkt thermisch, gegebenenfalls in Gegenwart von katalytischen Mengen Triphenylphosphin, oder mit stöchiometrischen Mengen Triethylamin zu V dehydrochlorieren. Anschließend wird V durch Destillation im Hochvakuum gereinigt.

Es ist bereits bekanntgeworden, daß man im Aroylrest halogenierte Aroylessigester erhält, wenn man die entsprechenden halogenierten Aroylhalogenide mit Malonester in Gegenwart von Magnesiumethylat zu den Aroylmalonestern umsetzt und diese unter selektiver Abspaltung einer Alkoxycarbonylgruppe zu den Aroylessigestern verseift und decarboxyliert (DE-OS 3 142 854).

Dieses Verfahren weist jedoch eine Reihe von Nachteilen auf. So wird beispielsweise durch weitergehende Verseifung und Decarboxylierung ein mehr oder weniger großer Anteil Acetophenon gebildet.

Verwendet man 2,4-Dichlor-5-fluor-benzoylchlorid (1) als Ausgangstoff, so kann der Reaktionsablauf durch das folgende Formelschema wiedergegeben werden :

(Siehe Formelschema Seite 3 f.)

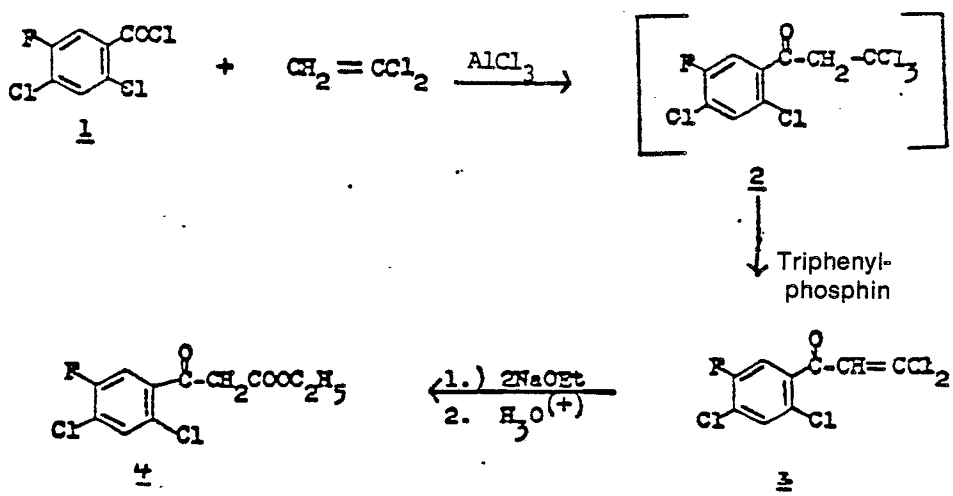


1 2 3 4

Der Aroylessigester (4) dient als Zwischenprodukt zur Synthese des antibakteriellen Breitspektrumchemotherapeutikums Ciprofloxacin (DE-OS 3 142 854), das auf folgendem Wege erhalten wird :

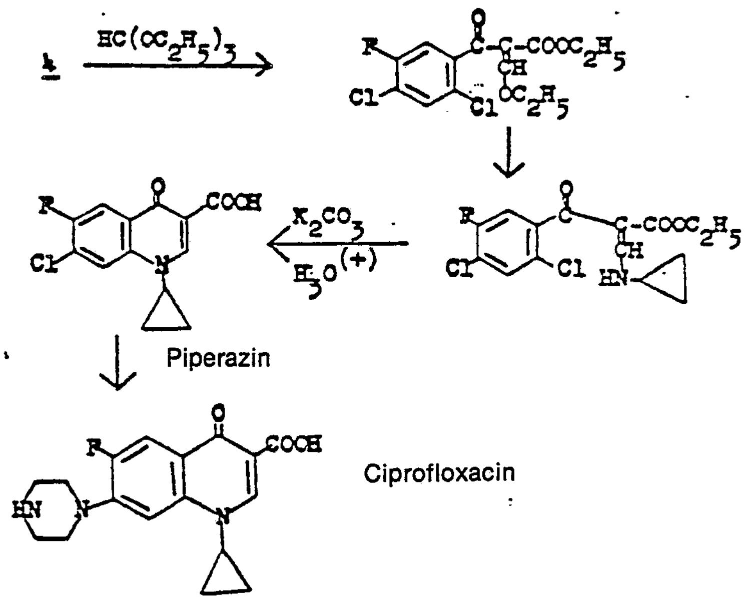


Ciprofloxacin

In der Formel (I) steht R vorzugsweise für Methyl oder Ethyl.

Die erfindungsgemäß verwendbaren fluor- und chlorhaltigen Benzoylchloride oder die entsprechenden Carbonsäuren sind bekannt, z. B. aus DE-OS 3 142 856.

Als Beispiele seien genannt :

2,4-Dichlor-5-fluor-benzoylchlorid, 2,4,5-Trifluorbenzoylchlorid, 2,3,4,5-Tetrafluor-benzoylchlorid, 2,4,5-Trichlorbenzoylchlorid, 2,3,4,5-Tetrachlor-benzoylchlorid. 2,4,5-Trifluor-3-chlorbenzoylchlorid.

Die Umsetzung der halogenierten Benzoylchloride II mit Dichlorethylen III zu IV wird vorzugsweise in einem inerten Verdünnungsmittel vorgenommen. In Frage kommen Schwefelkohlenstoff und Methylenchlorid.

Die Umsetzung wird bei Temperaturen zwischen 10 und 40 °C vorzugsweise zwischen 20 und 30 °C durchgeführt. Es wird bevorzugt bei Normaldruck gearbeitet.

Bei der Durchführung des erfindungsgemäßen Verfahrens setzt man auf 1 Mol Carbonsäurechlorid II 1 Mol wasserfreies Aluminiumchlorid ein. Vorteilhaft ist ein Überschuß von 5 bis 10 %.

Für 1 Mol Benzoylchlorid II wird 1 Mol Dichlorethylen III benötigt. Es hat sich als vorteilhaft erwiesen, wenn man auf 1 Mol (II) 1,5 bis 3 Mol Dichlorethylen verwendet.

Zur Dehydrochlorierung werden die Aryl-trichlorethyl-ketone IV mit katalytischen Mengen (ca. 0,1 g auf 100 g Rohprodukt) Triphenylphosphin bis zur Beendigung der Gasentwicklung auf 80 bis 180 °C, vorzugsweise 120 bis 150 °C erhitzt. Anschließend kann direkt im Hochvakuum destilliert werden, wobei die gebildeten Aryl-dichlorvinylketone V in hoher Ausbeute und Reinheit erhalten werden.

Die HCl-Abspaltung kann auch mit stöchiometrischen Mengen einer tertiären Base wie z. B. Triethylamin in einem Lösungsmittel wie z. B. Methylenchlorid oder Toluol durchgeführt werden. Nach Beendigung der bereits bei Raumtemperatur ablaufenden Reaktion wird mit Wasser gewaschen, mit Natriumsulfat getrocknet und nach dem Abdestillieren des Lösungsmittels das Vinylketon V durch

Destillation im Vakuum gereinigt.

Zur Umsetzung der Aryl-dichlorvinylketone V mit Alkoholat werden 2 g-Atome Natrium oder 2 Mol Natriumalkoholat in wasserfreiem Alkohol, bevorzugt Methyl- oder Ethylalkohol gelöst. Dann wird unter guter Kühlung 1 Mol Dichlorvinylketon eingetragen. Wenn die exotherme Reaktion beendet ist, wird noch ca. 1 Stunde bei Raumtemperatur gerührt und der Alkohol im Vakuum entfernt. Man nimmt in Methylenchlorid auf, schüttelt mit verdünnter Schwefelsäure, isoliert den Aroylessigester und reinigt durch Destillation im Vakuum.

Beispiel 1

2,4-Dichlor-5-fluor-benzoylessigsäureethylester

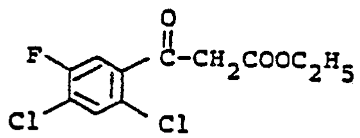

Eine Lösung von 4,6 g Natrium in 80 ml absolutem Ethanol wird unter Kühlung (Eis/Methanol) und Rühren bei 0-5 °C portionsweise mit 28,8 g (0,1 Mol) 2,2-Dichlorvinyl-(2,4-dichlor-5-fluor-phenyl)-keton versetzt. Nach dem Abklingen der exothermen Reaktion wird noch 30 Minuten bei Raumtemperatur gerührt, das Lösungsmittel im Vakuum abgezogen und der Rückstand in 100 ml Methylenchlorid aufgenommen. Man schüttelt mit 100 ml 2n Schwefelsäure gut durch, trennt die Methylenchloridlösung im Scheidetrichter ab und extrahiert noch dreimal mit jeweils 50 ml Methylenchlorid nach. Die vereinigten Methylenchlorid-Lösungen werden mit 100 ml gesättigter Kochsalzlösung gewaschen und über Natriumsulfat getrocknet. Dann wird das Lösungsmittel im Vakuum abdestilliert. Der braune, ölige Rückstand kristallisiert beim Stehen. Die Feinvakuumdestillation liefert 22,3 g (79,9 %) 2,4-Dichlor-5-fluor-benzoylessigsäureethylester vom Siedepunkt 125-126 °C/0,16 mbar. Farblose Kristalle vom Schmelzpunkt 44-45 °C.

Das als Ausgangsmaterial verwendete 2,2-Dichlorvinyl-(2,4-dichlor-5-fluor-phenyl)-keton wird wie folgt erhalten :

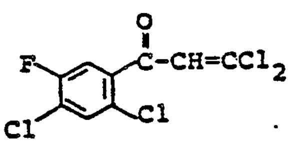

Eine Suspension von 84 g wasserfreiem Aluminiumchlorid in 300 ml Methylenchlorid wird unter Eiskühlung und Rühren nacheinander tropfenweise mit 136,5 g (0,6 Mol) 2,4-Dichlor-5-fluor-benzoylchlorid und mit 97 g (1 Mol) 1,1-Dichlorethylen versetzt, wobei die Temperatur 30 °C nicht überschreiten soll. Man rührt 3 Stunden bei Raumtemperatur, gießt auf ein Gemisch von ca. 600 g Eis und 60 ml konz. Salzsäure, trennt die organische Phase ab und extrahiert dreimal mit 100 ml Methylenchlorid nach. Die Methylenchloridlösung wird mit 100 ml Wasser gewaschen, mit Natriumsulfat getrocknet und das Lösungsmittel im Vakuum abdestilliert. Den Rückstand (218,8 g) erhitzt man mit 0,34 g Triphenylphosphin bis zur Beendigung der zunächst lebhaften HCl-Entwicklung auf 130-140 °C (ca. 3/4 Stunden). Die Destillation im Feinvakuum liefert 167,1 g (96,4 %) 2,2-Dichlorvinyl-(2,4-dichlor-5-fluorphenyl)-keton vom Siedepunkt 121-123 °C/0,13 mbar, Schmelzpunkt 64-65 °C.

Beispiel 2

2,4-Dichlor-5-fluor-benzoylessigsäuremethylester

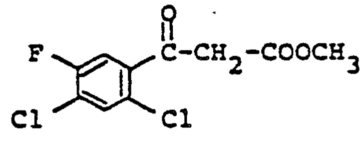

Eine Lösung von 9,2 g Natrium in 100 ml absolutem Methanol wird unter Kühlung (Eis/$CH_3OH$) und Rühren bei 0-10 °C portionsweise mit 57,6 g (0,2 mol) 2,2-Dichlorvinyl-(2,4-dichlor-5-fluor-phenyl)-keton versetzt. Man rührt die orangebraune Suspension 40 Minuten bei Raumtemperatur und destilliert anschließend das Lösungsmittel im Vakuum ab. Den Rückstand nimmt man in ca. 200 ml Methylenchlorid und 100 ml 2n Schwefelsäure auf, schüttelt gut durch, trennt die Phasen und extrahiert die schwefelsaure Lösung dreimal mit jeweils 80 ml Methylenchlorid nach. Die vereinigten $CH_2Cl_2$-Lösungen werden mit 50 ml gesättigter Kochsalzlösung gewaschen, mit Natriumsulfat getrocknet und das Methylenchlorid im Vakuum abgezogen. Man erhält 53 g braunes Festprodukt vom Schmelzpunkt 78-83 °C. Die Destillation

im Feinvakuum liefert 48 g (90,6 %) 2,4-Dichlor-5-fluor-benzoylessig-säuremethylester vom Siedepunkt 134-137 °C/0,15-0,17 mbar. Die farblosen Kristalle schmelzen bei 77-79 °C. (Destillationsrückstand : 4,1 g dunkelbraunes z. T. kristallisierendes Öl). Die Reinigung des Rohprodukts ist auch durch Umkristallisation aus Leichtbenzin/Tonsil möglich.

Beispiel 3

2,4,5-Trifluor-benzoylessigsäureethylester

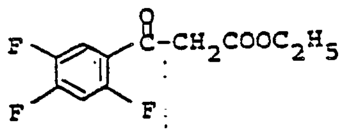

Eine Lösung von 3,85 g Natrium in 65 ml absolutem Ethanol wird unter Kühlung (Eis/Methanol) und Rühren bei 5-10 °C tropfenweise mit 21,2 g 2,2-Dichlorvinyl-(2,4,5-trifluorphenyl)-keton versetzt. Nach dem Abklingen der exothermen Reaktion wird noch 30 Minuten bei Raumtemperatur gerührt, das Lösungsmittel im Vakuum abgezogen und der Rückstand in 100 ml Methylenchlorid aufgenommen. Man schüttelt mit 50 ml 2n Schwefelsäure gut durch, trennt die Methylenchlorid-Phase im Scheidetrichter ab und extrahiert noch dreimal mit jeweils 50 ml Methylenchlorid nach. Die vereinigten $CH_2Cl_2$-Lösungen werden mit 100 ml gesättigter Kochsalzlösung gewaschen und über Natriumsulfat getrocknet. Dann wird das Lösungsmittel im Vakuum abdestilliert und der ölige Rückstand im Feinvakuum fraktioniert. Es werden 16,8 g (82,2 %) 2,4,5-Trifluor-benzoylessigsäureethylester vom Siedepunkt 98-100 °C/0,15 mbar erhalten. Der Schmelzpunkt beträgt 63-64 °C.

Das als Ausgangsmaterial verwendete 2,2-Dichlorvinyl-(2,4,5-trifluorphenyl)-keton wird wie folgt erhalten :

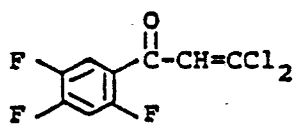

Eine Suspension von 18,5 g wasserfreiem Aluminiumchlorid in 75 ml trockenem Methylenchlorid wird unter Eiskühlung und Rühren nacheinander tropfenweise mit 25 g 2,4,5-Trifluorbenzoylchlorid in 10 ml Methylenchlorid und mit 37,5 g 1,1-Dichlorethylen versetzt, wobei die Temperatur 15 °C nicht überschreiten soll. Man rührt 2 Stunden bei ca. 15 °C, läßt über Nacht auf Raumtemperatur kommen, gießt auf ein Gemisch von 150 g Eis und 15 ml konz. Salzsäure, trennt die organische Phase ab und extrahiert mit Methylenchlorid nach. Die Methylenchloridlösung wird mit Wasser gewaschen, mit Natriumsulfat getrocknet und das Verdünnungsmittel im Vakuum abdestilliert. Den Rückstand (44 g) erhitzt man mit 0,1 g Triphenylphosphin bis zur Beendigung der zunächst lebhaften Gasentwicklung auf 130-140 °C (ca. 45 Minuten). Die Destillation im Vakuum liefert 23,8 g (72,8 %) 2,2-Dichlorvinyl-(2,4,5-trifluorphenyl)-keton vom Siedepunkt 132-134 °C/14 mbar.

**Patentansprüche**

1. Verfahren zur Herstellung von halogenierten Aroylessigestern der Formel I

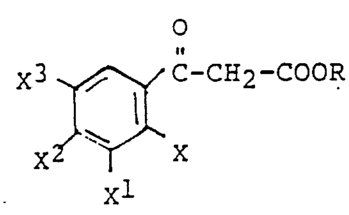

(I)

in welcher
R Alkyl mit 1-6 Kohlenstoffatomen,
X Halogen, vorzugsweise Chlor oder Fluor,
$X^1$ Wasserstoff oder Halogen, vorzugsweise Fluor,
$X^2$ Halogen, vorzugsweise Chlor oder Fluor, und
$X^3$ Wasserstoff oder Halogen, vorzugsweise Fluor, bedeutet,
dadurch gekennzeichnet, daß man Benzoylchloride der Formel II

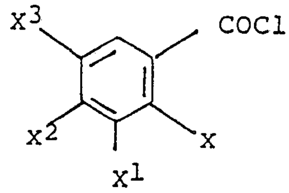

(II)

in der X, $X^1$, $X^2$ und $X^3$ die oben angegebene Bedeutung haben, in Gegenwart von wasserfreiem Aluminiumchlorid mit Dichlorethylen umsetzt, die entstehenden Aryltrichlorethylketone dehydrochloriert und die entstehenden Aryldichlorvinylketone mit Alkoholat und anschließend mit einer wäßrigen Säure behandelt.

2. Verfahren nach Anspruch 1, dadurch gekennzeichnet, daß man die Umsetzung der Benzoylchloride mit Dichlorethylen bei Temperaturen von 10 bis 40 °C, vorzugsweise 20 bis 30 °C durchführt.

3. Verfahren nach Anspruch 1, dadurch gekennzeichnet, daß man die Umsetzung der Benzoylchloride mit Dichlorethylen unter Verwendung von 1 Mol wasserfreiem Aluminiumchlorid pro Mol Benzoylchlorid durchführt.

4. Verfahren nach Anspruch 1, dadurch gekennzeichnet, daß man die Umsetzung der Benzoylchloride mit Dichlorethylen unter Verwendung von 1,5-3 Mol Dichlorethylen pro Mol Benzoylchlorid durchführt.

5. Verfahren nach Anspruch 1, dadurch gekennzeichnet, daß man die Dehydrochlorierung der Aryltrichlorethylketone in Gegenwart katalytischer Mengen Triphenylphosphin durchführt.

6. Verfahren nach Anspruch 1, dadurch gekennzeichnet, daß man die Dehydrochlorierung der Aryltrichlorethylketone in Gegenwart stöchiometrischer Mengen einer tertiären Base in einem Lösungsmittel durchführt.

7. Verfahren nach Anspruch 1, dadurch gekennzeichnet, daß man die Aryldichlorvinylketone mit 2 Mol Natriumalkoholat in wasserfreiem Alkohol und anschließend mit verdünnter wäßriger Schwefelsäure behandelt.

8. Verfahren nach Anspruch 5, dadurch gekennzeichnet, daß man die Dehydrochlorierung bei Temperaturen von 80 bis 180 °C durchführt.

9. Verfahren nach Anspruch 1, dadurch gekennzeichnet, daß man auf 1 Mol Carbonsäurechlorid II 1 Mol wasserfreies Aluminiumchlorid einsetzt.

10. Verfahren nach Anspruch 1, dadurch gekennzeichnet, daß man die Reaktion in Schwefelkohlenstoff oder Methylenchlorid durchführt.

**Claims**

1. Process for the preparation of halogenated aroylacetic acid esters of the formula I

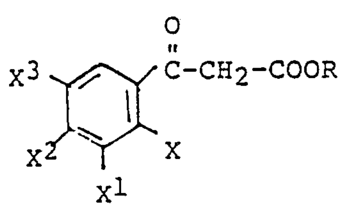

(I)

in which

R denotes alkyl with 1-6 carbon atoms,
X denotes halogen, preferably chlorine or fluorine,
$X^1$ denotes hydrogen or halogen, preferably fluorine,
$X^2$ denotes halogen, preferably chlorine or fluorine, and
$X^3$ denotes hydrogen or halogen, preferably fluorine,

characterised in that benzoyl chlorides of the formula II

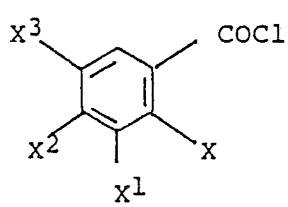

(II)

in which X, $X^1$, $X^2$ and $X^3$ have the abovementioned meaning, are reacted with dichloroethylene in the presence of anhydrous aluminium chloride, the aryl trichloroethyl ketones formed are dehydrochlorinated

and the aryl dichlorovinyl ketones formed are treated with an alcoholate and then with an aqueous acid.

2. Process according to Claim 1, characterised in that the reaction of the benzoyl chlorides with dichloroethylene is carried out at temperatures of 10 to 40 °C, preferably 20 to 30 °C.

3. Process according to Claim 1, characterised in that the reaction of the benzoyl chlorides with dichloroethylene is carried out using 1 mole of anhydrous aluminium chloride per mole of benzoyl chloride.

4. Process according to Claim 1, characterised in that the reaction of the benzoyl chlorides with dichloroethylene is carried out using 1.5-3 moles of dichloroethylene per mole of benzoyl chloride.

5. Process according to Claim 1, characterised in that the dehydrochlorination of the aryltrichloroethyl ketones is carried out in the presence of catalytic amounts of triphenylphosphine.

6. Process according to Claim 1, characterised in that the dehydrochlorination of the aryltrichloroethyl ketones is carried out in the presence of stoichiometric amounts of a tertiary base in a solvent.

7. Process according to Claim 1, characterised in that the aryldichlorovinyl ketones are treated with 2 moles of sodium alcoholate in an anhydrous alcohol and then with dilute aqueous sulphuric acid.

8. Process according to Claim 5, characterised in that the dehydrochlorination is carried out at temperatures of 80 to 180 °C.

9. Process according to Claim 1, characterised in that 1 mole of anhydrous aluminium chloride is employed per mole of carboxylic acid chloride II.

10. Process according to Claim 1, characterised in that the reaction is carried out in carbon disulphide or methylene chloride.

**Revendications**

1. Procédé de fabrication d'esters aroylacétiques halogénés de formule I :

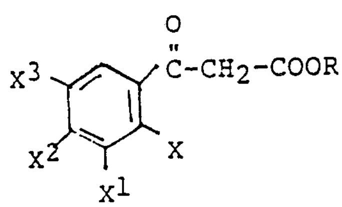

(I)

dans laquelle

R représente un alcoyle ayant 1 à 6 atomes de carbone,

X de l'halogène, de préférence du chlore ou du fluor,

$X^1$ de l'hydrogène ou de l'halogène, de préférence du fluor,

$X^2$ de l'halogène, de préférence du chlore ou du fluor et

$X^3$ de l'hydrogène ou de l'halogène, de préférence du fluor,

caractérisé en ce qu'on fait réagir des chlorures de benzoyle de formule II

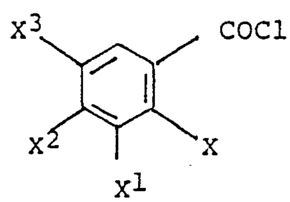

(II)

dans laquelle X, $X^1$, $X^2$ et $X^3$ ont la signification indiquée plus haut, en présence de chlorure d'aluminium anhydre avec du dichloréthylène, on déshydrochlorure les aryltrichloréthylcétones obtenues et on traite les aryldichlorovinylcétones obtenues avec un alcoolate et ensuite avec un acide aqueux.

2. Procédé selon la revendication 1, caractérisé en ce qu'on effectue la réaction des chlorures de benzoyle avec le dichloréthylène à des températures de 10 à 40 °C, de préférence de 10 à 30 °C.

3. Procédé selon la revendication 1, caractérisé en ce qu'on effectue la réaction des chlorures de benzoyle avec le dichloréthylène en utilisant 1 mole de chlorure d'aluminium par mole de chlorure de benzoyle.

4. Procédé selon la revendication 1, caractérisé en ce qu'on exécute la réaction des chlorures de benzoyle avec le dichloréthylène en utilisant 1,5 à 3 moles de dichloréthylène par mole de chlorure de benzoyle.

5. Procédé selon la revendication 1, caractérisé en ce qu'on exécute la déshydrochloruration des aryltrichloréthylcétones en présence de quantités catalytiques de triphénylphosphine.

6. Procédé selon la revendication 1, caractérisé en ce qu'on exécute la déshydrochloruration des

aryltrichloréthylcétones en présence de quantités stoechiométriques d'une base tertiaire dans un solvant.

7. Procédé selon la revendication 1, caractérisé en ce qu'on traite les aryldichlorovinylcétones avec 2 moles d'alcoolate de sodium dans de l'alcool anhydre et ensuite avec de l'acide sulfurique aqueux dilué.

8. Procédé selon la revendication 5, caractérisé en ce qu'on exécute la déshydrochloruration à des températures de 80 à 180 °C.

9. Procédé selon la revendication 1, caractérisé en ce qu'on utilise pour 1 mole de chlorure d'acide carboxylique II 1 mole de chlorure d'aluminium anhydre.

10. Procédé selon la revendication 1, caractérisé en ce qu'on effectue la réaction dans du sulfure de carbone ou dans du chlorure de méthylène.